# EUROPÄISCHE PATENTSCHRIFT

(11) **EP 2 946 831 B1**
(45) Veröffentlichungstag und Bekanntmachung des Hinweises auf die Patenterteilung: **20.02.2019**
(21) Anmeldenummer: 15164248.5
(22) Anmeldetag: 20.04.2015
(51) Int. Cl.: B01J 31/40, C07D 301/12

(54) **MEMBRANGESTÜTZTE KATALYSATORABTRENNUNG BEI DER EPOXIDIERUNG VON CYCLISCHEN, UNGESÄTTIGTEN C12-VERBINDUNGEN ZUM BEISPIEL CYCLODODECEN (CDEN)**
MEMBRANE-SUPPORTED CATALYST SEPARATION IN THE EPOXIDATION OF CYCLIC, UNSATURATED C12 COMPOUNDS FOR EXAMPLE CYCLODODECENE (CDEN)
SÉPARATION DE CATALYSEUR ASSISTÉE PAR MEMBRANE LORS DE L'ÉPOXYDATION DE LIAISONS C12 CYCLIQUES, INSATURÉS PAR EXEMPLE CYCLODODÉCANE (CDEN)

(30) Priorität: 19.05.2014 DE 102014209421
(43) Veröffentlichungstag der Anmeldung: 25.11.2015
(73) Patentinhaber: Evonik Degussa GmbH, 45128 Essen (DE)
(72) Erfinder: Meier, Ralf, 44265 Dortmund (DE); Micoine, Kévin, 45701 Herten (DE); Kreis, Peter, 44227 Dortmund (DE); Gluth, Frederik, 40474 Düsseldorf (DE); Priske, Markus, Mobile, AL, AL Alabama 36609 (US)

(56) Entgegenhaltungen:
- WO-A1-2011/067054
- M. N. TIMOFEEVA ET AL: "Epoxidation of Cycloolefins with Hydrogen Peroxide in the Presence of Heteropoly Acids Combined with Phase Transfer Catalyst.", CHEMINFORM, Bd. 34, Nr. 41, 14. Oktober 2003 (2003-10-14), Seiten 480-486, XP055200314, ISSN: 0931-7597, DOI: 10.1002/chin.200341107
- T.C.O. MAC LEOD ET AL: "An environmentally friendly triphasic catalytic system: Mn(salen) occluded in membranes based on PDMS/PVA", APPLIED CATALYSIS. B, ENVIRONMENTAL, Bd. 100, Nr. 1-2, 11. Oktober 2010 (2010-10-11), Seiten 55-61, XP055200286, ISSN: 0926-3373, DOI: 10.1016/j.apcatb.2010.07.010
- SANKHANILAY ROY CHOWDHURY ET AL: "Recovery of Homogeneous Polyoxometallate Catalysts from Aqueous and Organic Media by a Mesoporous Ceramic Membrane without Loss of Catalytic Activity", CHEMISTRY - A EUROPEAN JOURNAL., Bd. 12, Nr. 11, 3. April 2006 (2006-04-03) , Seiten 3061-3066, XP055200347, WEINHEIM, DE ISSN: 0947-6539, DOI: 10.1002/chem.200501021

## Beschreibung

Die vorliegende Erfindung betrifft ein Verfahren zur Abtrennung homogener Katalysatoren aus organischen Reaktionsmischungen.

Verfahren zur Rückgewinnung von Katalysatoren aus Reaktionsmischungen sind für die Wirtschaftlichkeit industrieller chemischer Prozesse von grundsätzlicher Bedeutung, da erst diese Verfahren die Wiederverwertung von häufig teurem Katalysatormaterial möglich machen. Die Rückgewinnung von Katalysatoren stellt den Fachmann dabei insbesondere bei der homogenen Katalyse vor besondere Herausforderungen. Da bei der homogenen Katalyse der Katalysator und die Reaktanden in derselben Phase vorliegen, ist es meist unmöglich, den Katalysator durch einfache physikalische Trennverfahren, wie beispielsweise Zentrifugation oder herkömmliche Filtration, von den Reaktanden zu trennen.

Eine Möglichkeit zur Rückgewinnung homogener Katalysatoren bietet die Nanofiltration. Die Nanofiltration ist ein druckgetriebenes Membranverfahren, bei dem eine spezielle Membran zur Abtrennung bestimmter gelöster Komponenten aus einer flüssigen Phase dient. Die Selektivität der Membran kann dabei auf unterschiedlichen Mechanismen beruhen. Beim Größenausschlussmechanismus werden die zurückgehaltenen, gelösten Komponenten auf Grund von sterischen Effekten daran gehindert, die Membran zu durchdringen. Die Abtrennung hängt also von der Größe der gelösten Komponenten und der mittleren Porengröße der Membran ab. Meist wird die Membran dabei durch das Molekulargewicht der zurückgehaltenen Komponenten charakterisiert. Bei einem elektrostatischen Mechanismus ergibt sich die Selektivität der Membran aus der Oberflächenladung der Membran und der Ladung der gelösten Komponenten. Bei gleichem Vorzeichen der Ladung kommt es zu einer elektrostatischen Abstoßung und somit zum Rückhalt der gelösten Komponenten. Somit ist es beispielsweise auch möglich, die Nanofiltration zur Abtrennung von Schwermetallionen aus wässrigen Lösungen einzusetzen. Schließlich kann die Trennung auch darauf beruhen, dass die Membran eine eigene Phase bildet, in der sich die Bestandteile des zu trennenden Gemisches lösen. Die Trennung erfolgt dann auf Grund unterschiedlicher Löslichkeit und unterschiedlicher Diffusionsgeschwindigkeit der Komponenten. Die resultierenden Transportgeschwindigkeiten einzelner Komponenten können sich dabei so stark unterscheiden, dass diese von einander abgereichert bzw. getrennt werden. Die von einer Membran genutzten Trenneffekte sind mithin deutlich komplexer als der rein mechanisch wirkende Siebeffekt, den sich Filter zu Nutze machen.

Die Membrantrennung findet beispielsweise Anwendung bei der Wasser- und Abwasseraufbereitung. Ein Problem bei der Anwendung der Nanofiltration in anderen Milieus ist häufig die mangelnde Stabilität der Membran in anderen als in wässrigen Lösungen. Insbesondere die Stabilität der Membran gegenüber organischen Lösungsmitteln ist oft unzureichend. Darüber hinaus macht sich die Membran zur Erledigung ihrer Trennaufgabe die unterschiedlichsten Wechselwirkungen mit dem zu trennenden Medium zu nutze. Deswegen ist die Auswahl eines für die jeweilige Trennaufgabe geeigneten Membranmaterials alles andere als trivial.

Chowdhury et al. setzen bei der Epoxidierung von Olefinen für die Rückgewinnung von homogenen Katalysatoren aus organischen Lösungsmitteln eine Keramikmembran ein:
S. R. Chowdhury et al., "Recovery of homogeneous polyoxometallate catalysts from aqueous and organic media by a mesoporous ceramic membrane without loss of catalytic activity", Chemistry-A European Journal, 2006, 12. Jg., Nr. 11, S. 3061-3066

Eine Membran, die sich in besonderer Weise für den Einsatz in Gegenwart von epoxidierten cyclischen, ungesättigten C12-Verbindungen mit Wasserstoffperoxid eignet, wurde bisher nicht identifiziert.

Es ist Aufgabe der vorliegenden Erfindung, ein Membranmaterial zur Abtrennung von homogenen Katalysatorsystemen anzugeben, das in Reaktionsgemischen zur Epoxidierung von cyclischen, ungesättigten C12-Verbindungen mit Wasserstoffperoxid eingesetzt werden kann.

Verfahren zur Epoxidierung von cyclischen C12-Verbindungen unter Verwendung homogener Katalysatoren sind beispielsweise aus Angew. Chem. (1991), 103, 1706-1709, EP1205474, EP1411050 und EP1411051 bekannt.

Ein besonderer Schwerpunkt der vorliegenden Erfindung liegt dabei auf der Abtrennung von homogenen Übergangsmetallkatalysatorsystemen, die mit Hilfe eines Phasentransferreagenz in der organischen Phase gelöst werden. Als Phasentransferreagenz kommen dabei insbesondere tertiäre und quartäre Ammoniumverbindungen zum Einsatz. Ein speziell auf dieses Katalysatorsystem abgestimmtes Nanofiltrationsverfahren ist bislang nicht bekannt.

Umfangreiche Forschungstätigkeiten haben zu der Erkenntnis geführt, dass zur Abtrennung eines solchen Katalysatorsystems aus dem Reaktionsgemisch einer Epoxidierung von cyclischen, ungesättigten C12-Verbindungen sich solche Membranen eignen, die als trennaktive Schicht vernetzte Siliconacrylate und/oder Polydimethylsiloxan und/oder Polyimid enthalten. Diese drei Membranmaterialien können jeweils einzeln oder gemeinsam in der trennaktiven Schicht enthalten sein.

Gegenstand der Erfindung ist mithin ein Verfahren zur Abtrennung eines homogenen Katalysatorsystems aus einem Reaktionsgemisch, bei welchem das Reaktionsgemisch an einer zumindest eine trennaktive Schicht umfassenden Membran dergestalt getrennt wird, dass das homogene Katalysatorsystem zumindest teilweise im Retentat der Membran angereichert wird, wobei das Reaktionsgemisch zumindest eine teilweise epoxidierte cyclische, ungesättigte Verbindung mit zwölf Kohlenstoffatome enthält, und die trennaktive Schicht der Membran vernetzte Siliconacrylate und/oder Polydimethylsiloxan (PDMS) und/oder Polyimid umfasst.

Das Reaktionsgemisch umfasst zwei nicht oder schwer mischbaren flüssigen Phasen. Eine der Phasen enthält im Wesentlichen Wasser (wässrige Phase). Daneben kann diese Phase Wasserstoffperoxid, Katalysator, Phasentransferreagenz und Spuren der cyclischen, ungesättigten C12-Verbindung, der epoxidierten cyclischen, ungesättigten C12-Verbindung sowie dessen Folgeprodukt wie z.B. Diole enthalten. Die andere Phase (organische Phase) enthält typischerweise im Wesentlichen epoxidierte cyclische, ungesättigte C12-Verbindungen als Reaktionsprodukt der Epoxidierung. Daneben kann diese das Reaktionsgemisch die als Edukt eingesetzten unmodifizierten cyclischen, ungesättigten C12-Verbindungen sowie den Katalysator und das Phasentransferreagenz enthalten.

Bevor das Reaktionsgemisch der Membran zugeführt wird, erfolgt eine Trennung der beiden flüssigen Phasen. Diese kann beispielsweise mittels Phasentrennbehälter durchgeführt werden. Insofern wird das Reaktionsgemisch nicht als zweiphasiges, sondern als einphasiges Gemisch auf die Membran gegeben. Vorzugsweise wird die nichtwässrige (organische) Phase per Membran getrennt. Der Katalysator kann auch aus der wässrige Phase mittels Membrantechnik abtrennt werden. Mithin werden wässrige und organische Phase getrennt voneinander mit einer jeweiligen Membran von Katalysator bereinigt. Für die wässrige Phase kann eine andere Membran verwendet werden als für die organische Phase. Für die wässrige Phase wird vorzugsweise eines der folgenden Membranmaterialen verwendet: Polyamide, aromatische Polyamide, Polysulfone, Polyethersulfone, hydrophobierte Polyethersulfone, sulfonierte Polyethersulfone, Celluloseactat, Polypiperazin und Polyvinylidenfluorid. Für die Auftrennung der organischen Phase sollte hingegen eine Membran auf Basis von Siliconacrylat und/oder Polydimethylsiloxan (PDMS) und/oder Polyimid verwendet werden.

Sofern keine Phasentrennung des Reaktionsgemischs erfolgen sollte, sind dem Fachmann Maßnahmen zur Phasentrennung wie Erhöhung der Polarität der wässrigen Phase oder Änderung der Dichte einer Phase bekannt.

Unter dem Begriff cyclische, ungesättigte C12-Verbindungen werden im Rahmen dieser Erfindung deshalb sowohl die als Edukt eingesetzten cyclischen, ungesättigten C12-Verbindungen als auch die als Produkt erhaltenen epoxidierten cyclischen, ungesättigten C12-Verbindungen zusammengefasst. Sofern das Edukt genau eine Mehrfachbindung aufweist, ist das Produkt auf Grund der Epoxidierung der Mehrfachbindung eine gesättigte Verbindung.

Die erfindungsgemäß einsetzbaren Membranen bestehen nicht zwangsläufig ausschließlich aus den oben genannten trennaktiven Materialien, sie können auch noch weitere Materialien aufweisen. Insbesondere können die Membranen Stütz- oder Trägermaterialien aufweisen, auf denen die trennaktiven Schichten aufgebracht sind. Man spricht in einem solchen Fall von einer Verbund- oder Kompositmembran. Bei solchen Verbundmembranen liegt neben dem eigentlichen trennaktiven Material also noch ein Stützmaterial vor. Entsprechende Stützmaterialien offenbart zum Beispiel EP 0 781 116 A1.

Kommerziell erhältliche Membranen, die für das erfindungsgemäße Verfahren eingesetzt werden können, sind die unter der Typenbezeichnung MPF beziehungsweise SELRO erhältlichen Produkte der Firma Koch Membrane Systems, Inc., die Produkte der Firma Solsep B.V., die Produkte der Typenbezeichnung STARMEM von Grace/UOP, die Produkte der Typenbezeichnung PURAMEM und DURAMEM der Evonik Industries AG, die Produkte der Typenbezeichnung NANO-PRO von AMS Technologies Ltd. sowie die von der GMT Membrantechnik GmbH erhältlichen Membranen mit der Typenbezeichnung oNF-1, oNF-2 und NC-1.

Mit dem erfindungsgemäßen Verfahren ist es möglich, homogene Katalysatoren zur Epoxidierung von cyclischen, ungesättigten C12-Verbindungen, nach erfolgreicher Epoxidierung direkt aus dem Reaktionsgemisch zurückzugewinnen. Dies ermöglicht eine besonders wirtschaftliche Reaktionsführung, da der zurückgewonnene Katalysator für weitere Epoxidierungsreaktionen erneut eingesetzt werden kann.

Bei der cyclischen, ungesättigten Verbindung mit zwölf Kohlenstoffatomen handelt es sich bevorzugt um Cyclododecen (CDEN). CDEN wird beispielsweise durch selektive Hydrierung aus Cyclododecatrien gewonnen, welches wiederum aus der Trimerisierung von Butadien zugänglich ist. Durch die Epoxidierung wird Monoepoxy-Cyclododecan erhalten.

Das erfindungsgemäße Verfahren erlaubt die Abtrennung von homogenen Epoxidierungskatalysatorsystemen wie sie aus dem Stand der Technik bekannt sind. Es handelt sich hierbei um Übergangsmetallkatalysatoren, d.h. um Wolfram-, Molybdän- oder Vanadium-haltige Katalysatoren, wie sie beispielsweise in WO 00/44704 (A1) oder DE 30 27 349 A1 beschrieben sind.

Das Katalysatorsystem umfasst ein Derivat von Wolfram, Molybdän und/oder Vanadium. Als Derivat kommt ein Oxid, ein gemischtes Oxid, eine sauerstoffhaltige Säure, ein Salz einer sauerstoffhaltigen Säure, die Metallcarbonyle W(CO)₆ und Mo(CO)₆ ein Sulfid, ein Chlorid, ein Oxichlorid oder ein Stearat der Elemente Wolfram, Molybdän und/oder Vanadium in Betracht.

Geeignete Derivate sind beispielsweise die Metallcarbonyle W(CO)₆ oder Mo(CO)₆, die Oxide MoO₂, MoO₅, Mo₂O₃, MoO₃, WO₂, W₂O₅, WO₃, VO₂, V₂O₃, oder V₂O₅, die Sulfide WS₂ oder WS₃. Weitere Beispiele sind die Sauerstoffsäuren H₂WO₄ und H₂MoO₄ bzw. deren Alkali- oder Erdalkalisalze. Beispielsweise umfassen bekannte Epoxidierungskatalysatoren Wolframat oder Molybdat, insbesondere Natriumwolframat, Na₂WO₄, oder Natriummolybdat, Na₂MoO₄.

Diese Verbindungen werden meist in situ in die katalytisch aktive Verbindung umgewandelt. Dies erfolgt durch Umsetzung mit einem Derivat von Phosphor. Hierfür eignet sich ein Oxid, eine Sauerstoffsäure, ein Salz einer Sauerstoffsäure, ein Sulfid, ein Chlorid, ein Oxychlorid oder ein Fluorid von Phosphor.

Der Katalysator umfasst deshalb eine katalytisch aktive Verbindung, die durch Umsetzung eines Derivats von Wolfram, Molybdän oder Vanadium mit einem Derivat von Phosphor erhalten wird. In einer besonders bevorzugten Ausführungsform wird die katalytisch aktive Übergangsmetallverbindung in situ durch Umsetzung von Natriumwolframat mit Phosphorsäure gebildet.

Die beschriebenen Katalysatoren umfassen ein Phasentransferreagenz, mit dessen Hilfe der an sich wasserlösliche Katalysator in die organische C12-Phase überführt werden kann.

IDer Katalysator umfasst demnach ein Derivat von Wolfram, Molybdän oder Vanadium in Verbindung mit einem Phasentransferreagenz.

Phasentransferreagenzienwerden aus ternären und quartären Ammoniumverbindungen ausgewählt, wie sie beispielsweise in DE 30 27 349 A1 beschrieben sind.

Als ternäre Ammoniumverbindungen werden im Rahmen dieser Erfindung organische Ammoniumverbindungen bezeichnet, bei denen drei Valenzen des Stickstoffatoms organisch gebunden sind. Darunter fallen insbesondere Verbindungen der Summenformel NR₃, bei dem R organische Reste sind, Imin-Verbindungen der Formel R=NR, als auch N-alkylierte Heteroaromaten.

Ein Beispiel für ein geeignetes Phasentransferreagenz ist das unter dem Namen Alamin erhältliche Trioctylamin. Die Octylreste können zumindest teilweise durch Decylreste ersetzt worden sein. Das Amin ist ein bevorzugtes Phasentransferreagenz dieser Erfindung.

Als quartäre Ammoniumverbindungen werden im Rahmen dieser Erfindung organische Ammoniumverbindungen bezeichnet, bei denen alle vier Valenzen des Stickstoffatoms organisch gebunden sind. Darunter fallen insbesondere Verbindungen der Summenformel NR₄⁺X⁻, bei dem alle vier R organische Reste sind, Imin-Verbindungen der Formel R=NR₂⁺X⁻, als auch N-alkylierte Heteroaromaten, wobei X⁻ jeweils das zugehörige Anion ist.

Ein Beispiel für ein geeignetes Phasentransferreagenz ist das unter dem Namen Aliquat 336 erhältliche Tricaprylylmethylammoniumchlorid.

Als weiterhin geeignete Phasentransferreagenzien für die Durchführung von Epoxidierungsreaktionen haben sich sogenannte Esterquats erwiesen. Mit dem Begriff Esterquat werden im Rahmen dieser Erfindung quartäre Ammoniumverbindungen bezeichnet, die mindestens eine Carbonsäureestergruppe aufweisen.

In einer besonders bevorzugten Ausführungsform umfasst das Katalysatorsystem als quartäre Ammoniumverbindung deshalb ein Esterquat der Formel (I) 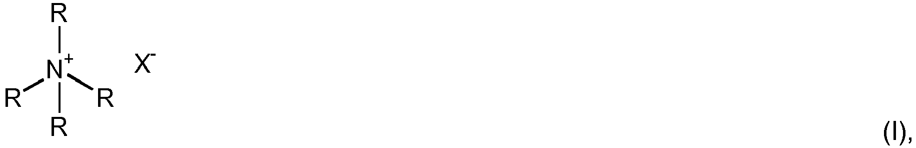 wobei die Substituenten R unabhängig voneinander gleiche oder verschiedene Alkyl- oder Alkoholgruppen mit 1 bis 4 Kohlenstoffatomen darstellen und mindestens einer der Substituenten R eine Alkoholgruppe mit 1 bis 4 Kohlenstoffatomen darstellt, die mit einer gesättigten oder ungesättigten Fettsäure mit 1 bis 30 Kohlenstoffatomen verestert ist, und wobei X⁻ für ein Gegenanion steht. Die ungesättigten Fettsäuren können einfach oder mehrfach ungesättigt sein. Besonders bevorzugt sind dabei gesättigte oder ungesättigte Fettsäuren mit 8 bis 20 Kohlenstoffatomen, am meisten bevorzugt 16 bis 18. Bevorzugte Gegenanionen X⁻ sind beispielsweise Chlorid, Cl⁻, und Methylsulfat, CH₃SO₄⁻.

Die Substituenten R gemäß Formel (I) stellen unabhängig voneinander gleiche oder verschiedene Alkyl- oder Alkoholgruppen mit 1 bis 4 Kohlenstoffatomen dar, wobei mindestens einer der Substituenten R eine veresterte Alkoholgruppe darstellt. Bevorzugt handelt es sich bei den Alkoholgruppen um einwertige Alkohole. Geeignete Alkylgruppen sind insbesondere Methyl, Ethyl, Propyl, iso-Propyl, n-Butyl, iso-Butyl, sec-Butyl und tert-Butyl. Geeignete Alkohole sind insbesondere sämtliche monohydroxylierte Derivate dieser Alkylgruppen, insbesondere Hydroxymethyl, 1-Hydroxyethyl, 2-Hydroxyethyl, 1-Hydroxypropyl, 2-Hydroxypropyl, 3-Hydroxypropyl, 1-Hydroxy-1-methyl-ethyl, 2-Hydroxy-1-methyl-ethyl, 1-Hydroxybutyl, 2-Hydroxybutyl, 3-Hydroxybutyl, 4-Hydroxybutyl, 1-Hydroxy-1-methyl-propyl, 2-Hydroxy-1-methyl-propyl, 3-Hydroxy-1-methyl-propyl, 1-Hydroxymethyl-propyl und 1-Hydroxymethyl-1-methyl-ethyl.

Als besonders geeignet haben sich dabei Ester einer quartären Ammoniumverbindung gemäß den Formeln Formeln (II) oder (III) 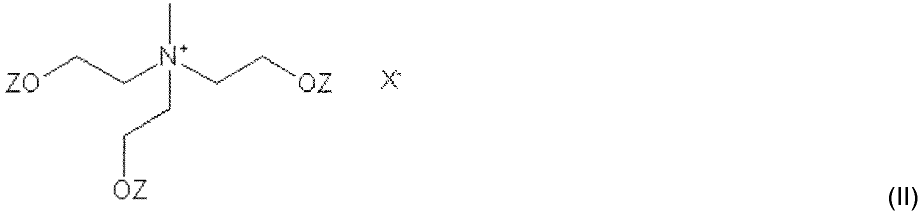 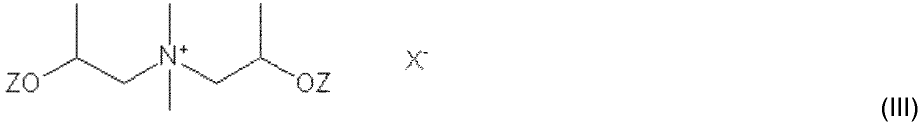 mit Z gleich einer oder mehreren, gesättigten oder ungesättigten Fettsäuren mit 1 bis 30 Kohlenstoffatomen erwiesen, wobei X⁻ für ein Gegenanion steht. Besonders bevorzugt sind dabei gesättigte oder ungesättigte Fettsäuren mit 8 bis 20 Kohlenstoffatomen, am meisten bevorzugt 16 bis 18.

Die beschriebenen Esterquats können hergestellt werden, indem die entsprechenden tertiären Alkoholamine, z.B. N-Methyldiisoproanolamin oder Triethanolamin, mit einer geeigneten Fettsäure verestert und anschließend mit einem geeigneten Alkylierungsmittel, z.B. Dimethylsulfat oder Diethylsulfat oder Methylchlorid, zu den quarternären Alkanolamninverbindungen umgesetzt werden.

Die Esterquats der Formeln (I) bis (III) liegen in der Regel als Mischungen verschiedener Ester vor, wobei sowohl die Fettsäuregruppe als auch die Anzahl der veresterten Fettsäuregruppen pro Alkanolaminverbindung variieren können. Esterquats werden deshalb anhand ihres Veresterungsgrades charakterisiert. Dies ist die mittlere Anzahl von veresterten Fettsäuren pro Alkanolaminverbindung. Bevorzugt liegt der Veresterungsgrad im Bereich von 1 bis 2,0 besonders bevorzugt von 1,2 bis 2,0 am meisten bevorzugt von 1,5 bis 1,95. Für die Synthese eines Esterquats mit einem gewünschten Veresterungsgrad wird die Alkanolaminverbindung mit einer dem Veresterungsgrad entsprechender Stoffmenge an Fettsäure umgesetzt. Die Umsetzung der Veresterung wird anhand der Säurezahl nach der Synthese überprüft. Bei Säurezahl kleiner als 5 g KOH pro g Lösung ist die Umsetzung hinreichend vollständig.

Bevorzugt wird eine Kompositmembran zur Abtrennung des Katalysators genutzt. Eine Kompositmembran umfasst ein Stützmaterial und ein darauf aufgebrachtes Membranmaterial als trennaktive Schicht. Das Stützmaterial unterscheidet sich mithin von dem trennaktiven Material. Es hat sich gezeigt, dass Kompositmembranen, die ein poröses Trägermaterial und eine durch Vernetzung von Siliconacrylaten oder Polydimethylsiloxanen hergestellte Schicht umfassen, sowohl äußerst beständig gegenüber dem beschriebenen Reaktionsgemisch sind, als auch zu einer hohen Ausbeute an rückgewonnenem Katalysator führen. Insbesondere eignen sich die beschriebenen Kompositmembranen zur Rückgewinnung von Katalysatoren, die ein Derivat von Wolfram, Molybdän oder Vanadium sowie die beschriebenen Esterquats als Phasentransferreagenz umfassen.

Die Wirkung der Kompositmembran beruht dabei vermutlich darauf, dass der Transport des Katalysators durch die Membran stärker gehindert wird als die restlichen Bestandteile des Reaktionsgemisches, insbesondere die cyclischen ungesättigten C12-Verbindungen treten schneller als der Katalysator durch die Membran. Eine besondere Rolle kommt hierbei der der auf vernetzten Siliconacrylaten oder Polydimethylsiloxanen basierenden Schicht zu, die vermutlich maßgeblich für die Beeinflussung des Diffusionsverhaltens ist und deshalb auch als trennaktive Schicht bezeichnet wird. Das Stützmaterial wird bei der Trennwirkung nur eine untergeordnete Rolle spielen, seine grundsätzliche Beteiligung an der Lösung der Trennaufgabe kann aber nicht ausgeschlossen werden; dafür sind die Vorgänge an einer Membran zu komplex.

Geeignete Kompositmembranen und deren Herstellung sind beispielsweise in DE19507584, EP1741481 und WO 2011/067054 A1 beschrieben.

Besonders geeignete Membranen sind dadurch gekennzeichnet, dass es sich bei den Siliconacrylaten um Verbindungen der Formel IV handelt 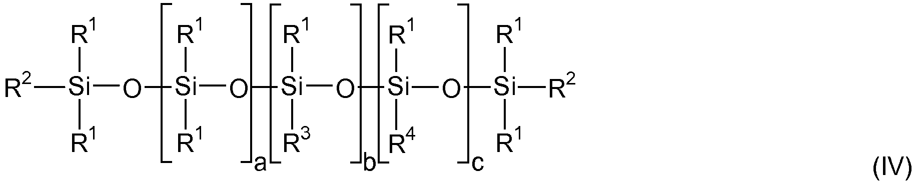 wobei
a = 1 bis 500, b = 1 bis 25 und c = 0 bis 20 ist,
die Substituenten R¹ unabhängig voneinander gleiche oder verschiedene Alkyl- oder Arylgruppen mit 1 bis 30 Kohlenstoffatomen darstellen, die gegebenenfalls mindestens eine Ether-, Ester-, Epoxy- und/oder Hydroxygruppe tragen können,
die Substituenten R² unabhängig voneinander gleiche oder verschiedene Substituenten aus der Gruppe R¹, R³ und R⁴ darstellen,
die Substituenten R³ unabhängig voneinander gleiche oder verschiedene Acrylatgruppen der Formeln V oder VI darstellen 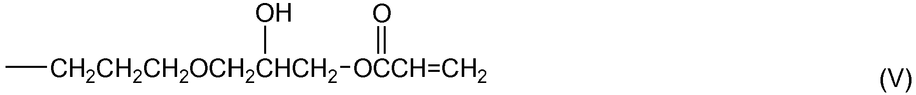 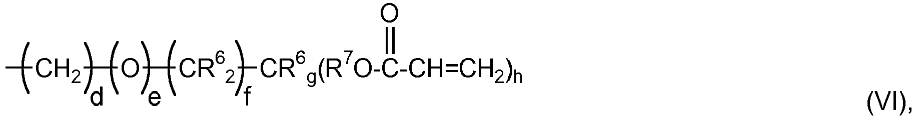
wobei d = 0 bis 12, e = 0 bis 1, f = 0 bis 12, g = 0 bis 2, h = 1 bis 3 und g + h = 3 ist, die Substituenten R⁶ unabhängig voneinander gleiche oder verschiedene Alkyl- oder Arylgruppen mit 1 bis 30 Kohlenstoffatomen oder Wasserstoff darstellen,
die Gruppen R⁷ gleiche oder verschiedene zweibindige Kohlenwasserstoffreste, bevorzugt -CR⁶₂-, insbesondere CH₂, darstellen,
die Substituenten R⁴ unabhängig voneinander gleiche oder verschiedene Polyethergruppen der Formel (VII) darstellen 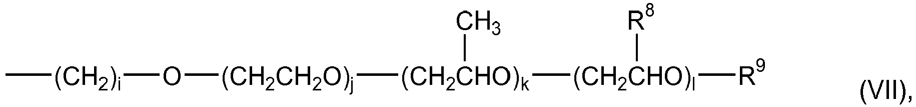
wobei i = 0 bis 12, j = 0 bis 50, k = 0 bis 50 und I = 0 bis 50 ist,
die Substituenten R⁸ gleiche oder verschiedene Alkyl- oder Arylgruppen mit 2 bis 30 Kohlenstoffatomen darstellen, und
R⁹ eine Alkyl-, Aryl- oder Acylgruppe mit 2 bis 30 Kohlenstoffatomen oder Wasserstoff darstellt.

Die verschiedenen Monomere der in den Formeln angegebenen Bausteine (Siloxanketten bzw. Polyoxyalkylenketten) können untereinander blockweise aufgebaut sein mit einer beliebigen Anzahl an Blöcken und einer beliebigen Sequenz oder einer statistischen Verteilung unterliegen. Die in den Formeln angegebenen Indizes sind als statistische Mittelwerte zu betrachten.

Besonders bevorzugt wird das trennaktive Membranmaterial durch Vernetzung von Siliconacrylaten der Formel IV in mehreren Schichten aufgebaut.

Eine solche Membran ist aus WO 2011/067054A1 bekannt.

Ein besonders vorteilhafte Familie von Membranen lässt sich herstellen, wenn die Herstellung durch Aushärtung einer Mischung verschiedener Siliconacrylate erfolgt. Durch die Wahl der Mischung lassen sich die Eigenschaften Trenngrenze, Vernetzungsgrad und Hydrophilie nahezu stufenlose in bisher nicht gekannten Bereichen einstellen.

Vorteilhafterweise weist die trennaktive Schicht selbst ein oder mehrere Schichten auf, welche durch Aushärtung einer Mischung verschiedener Siliconacrylate hergestellt wurden. Besonders vorteilhaft sind Mischungen bestehend aus zumindest folgenden Komponenten:
a) ein oder mehrere Siliconacrylate mit einem Siliziumgehalt von mehr als 29 Gewichts-%, bevorzugt ein oder mehrere Siliconacrylate der Formel IV mit einem Siliziumgehalt von mehr als 29 Gewichts-%, insbesondere ein oder mehrere Siliconacrylate der Formel IV mit b=c=0 und einem Siliziumgehalt von mehr als 29 Gewichts-%, und
b) ein oder mehrere Siliconacrylate mit einem Siliziumgehalt von weniger als 27,5 Gewichts-%, bevorzugt ein oder mehrere Siliconacrylate der Formel IV mit einem Siliziumgehalt von weniger als 27,5 Gewichts-%, insbesondere ein oder mehrere Siliconacrylate der Formel IV mit c>3 und einem Siliziumgehalt von weniger als 27,5 Gewichts-%,
wobei für die Komponente a) gilt, dass a = 25 bis 500, bevorzugt 25 bis 300, insbesondere 30-200 ist, b = 0 bis 15, bevorzugt 0 bis 8, insbesondere 0 ist und c = 0 bis 20, bevorzugt 0 bis 10, insbesondere 0 ist, mit der Maßgabe, dass bei b = 0 R² = R³ ist; und wobei für die Komponente b) gilt, dass a = 1 bis 24, bevorzugt 5 bis 20, besonders bevorzugt 10 bis 20 und insbesondere 10, 11, 12, 13, 14, 15, 16, oder 17 ist, b = 0 bis 25, bevorzugt 3 bis 10, besonders bevorzugt 3, 4, 5, 6, 7 oder 8 ist und c = 0 bis 20, bevorzugt 0 bis 10, besonders bevorzugt 0, 1, 2, 3 oder 4 ist, mit der Maßgabe, dass bei b = 0 R² = R³ ist.

Die Komponenten a) und b) liegen bevorzugt in einem Massenverhältnis von 10 zu 1 bis 1 zu 10 vor, insbesondere im Verhältnis 2 zu 8 bis 8 zu 2.

Verschiedene Siliconacrylate können durch ihren Siliziumanteil charakterisiert werden. Dieser ist umso kleiner, je mehr organische Substituenten an das Siloxangerüst gebunden sind und je länger diese Substituenten sind. Wird zur Beschichtung der Kompositmembran die oben beschriebene Mischung der Komponenten a) und b) eingesetzt, so werden als Komponente a) bevorzugt Siliconacrylate mit einem Siliziumgehalt von > 29 Gewichts-% und als Komponente b) bevorzugt Siliconacrylate mit einem Siliziumgehalt von < 27,5 Gewichts-% eingesetzt.

Die Siliconacrylate der Formel IV können durch Aushärtung mit einem Photoinitiator durch elektromagnetische Strahlung mit einer Wellenlänge von < 800 nm und/oder Elektronenstrahlen erhalten werden. Insbesondere erfolgt die Aushärtung durch UV-Strahlung bei einer Wellenlänge von < 400 nm.

Als Trägermembran eignen sich generell lösungsmittelbeständige poröse dreidimensionale Strukturen, wie z.B. Gewebevliese, Mikro- oder Ultrafiltrationsmembranen, oder Separatoren, wie z.B. Batterieseparatoren wie Separion® (Marke der Evonik Degussa GmbH) oder Solupor.

Besonders geeignete Trägermembranen bestehen aus Polyacrylnitril, Polyimid, Polyetheretherketon, Polyvinylidenfluorid, Polyamid, Polyamidimid, Polyethersulfon, Polybenzimidazol, sulfoniertes Polyetherketon, Polyethylen, Polypropylen oder Mischungen der genannten Materialien.

Als besonders geeignet haben sich Polyimidträgermembranen und Polyacrylnitrilträgermembranen erwiesen. Mithin wird bevorzugt eines der beiden Materialien als Trägermaterial innerhalb der Membran eingesetzt.

Die erfindungsgemäß einzusetzende Membran kann anhand einer Trennkurve charakterisiert werden. Sie wird im Rahmen dieser Erfindung anhand der Filtration in einem Modelsystem von in Toluol gelösten Polystyrololigomeren mit definierter Molekülmasse bei einer Temperatur von 30°C und einer transmembranen Druckdifferenz von 30 bar bestimmt. Mit diesem Modelsystem erzeugte Trennkurven dienen als Anhaltspunkt für die Membranauswahl. Aufgrund der unterschiedlichen Wechselwirkungen des realen Systems mit der Membran gegenüber dem Modellsystem werden die im Modellsystem bestimmten Trenngrenzen im realen System in der Regel nur mit einer gewissen Abweichung wiedergefunden.

Besonders bevorzugt weist die erfindungsgemäß einzusetzende Membran einen Verlauf der Trenngrenze für Molekulargewichte von mehr als 1000g/mol oberhalb von 97% Rückhalt und für Molekulargewichte von weniger als 300g/mol unterhalb von 80% Rückhalt.

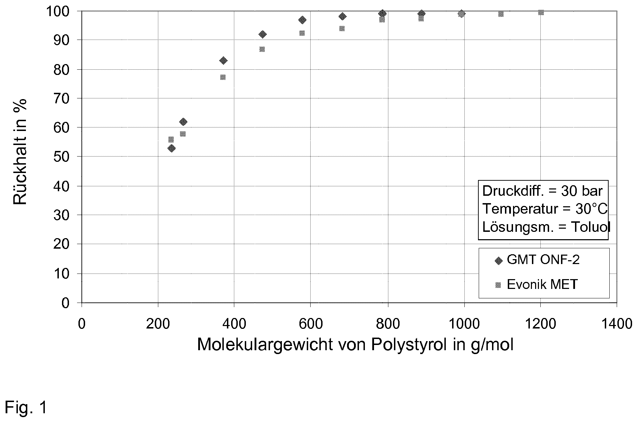

Die Trenngrenze einer besonders geeigneten Membran ist in Figur 1 graphisch dargestellt. Dabei wurde die Trenngrenze in etablierter Weise mit Polystyrol in Toluol bei einer Trenntemperatur von 30°C und einem Transmembrandruck von 30 bar ermittelt.

Die erfindungsgemäße Filtration des Reaktionsgemisches ist in der Regel ein druckgetriebener Prozess. Das bedeutet, dass entlang der Membran eine Druckdifferenz (transmembrane Druckdifferenz, auch "Transmembrandruck" genannt) angelegt wird, um den Durchtritt des Reaktionsgemisches durch die Membran zu forcieren. Bevorzugt beträgt der Transmebrandruck 5 bis 80 bar, besonders bevorzugt 10 bis 60 bar, insbesondere 30 bis 40 bar.

Die Temperatur während der Filtration kann in Abhängigkeit von der Temperaturstabilität der Membran und der Reaktionsmischung angepasst werden. Es ist von Vorteil, die Temperatur so hoch wie möglich zu wählen, um die Viskosität der Reaktionsmischung zu reduzieren und somit die Filtration zu beschleunigen. Bevorzugt wird die Filtration bei einer Temperatur von 25 bis 160 °C durchgeführt, besonders bevorzugt 40 bis 110 °C, insbesondere 60 bis 90°C.

Mit dem erfindungsgemäßen Verfahren wird das Katalysatorsystem im Retentat angereichert. Unter "Retentat" versteht der Membrantechniker den Abfluss von der Membran, der vor der Membran abgezogen wird. Das Material, welches die Membran überwindet, wird "Permeat" genannt und hinter der Membran abgezogen. Die Menge an zurückgehaltenem Katalysator kann dabei anhand der Menge an zurückgehaltenem Übergangsmetall nachgewiesen werden. Das erfindungsgemäße Verfahren erlaubt es insbesondere, mehr als 50 % des Übergangsmetalls, bezogen auf die Gesamtmenge an Übergangsmetall im Reaktionsgemisch vor der Filtration, im Retentat zurückzuhalten. Bevorzugt werden mehr als 70 % des Übergangsmetalls zurückgehalten, besonders bevorzugt mehr als 90 %. Falls ein Phasentransferreagenz eingesetzt wird, wird dieses in der Regel einen anderen Rückhalt erzielen als das Übergangsmetall. Dennoch wird es im Retentat angereichert. Deswegen wird das gesamte Katalysatorsystem zumindest teilweise im Retentat angereichert. Das Übergangsmetall kann durch ICP-MS (Massenspektroskopie mit induktiv gekoppeltem Plasma) oder RFA (Röntgenfluoreszenzanalyse) nachgewiesen werden.

Die Bestimmung von Wolfram und Phosphor erfolgt mittels ICP-OES mit einem Spektrometer der Firma Agilent. Da nur saure, wässrige Lösungen vermessen werden können, müssen die Proben vorher mittels Mikrowellenaufschluss (UltraClave der Firma MLS) aufgeschlossen werden.

Die Stickstoffbestimmung erfolgt durch eine oxidative Verbrennungsanalyse (bei 1050°C im Quarzrohr im Sauerstoffstrom) mit anschließender Chemolumineszenz-Detektion des aus NO in Gegenwart von Ozon gebildeten angeregten NO₂.

In einer Ausführungsform des Verfahrens findet die Filtration in einer speziellen Trennvorrichtung statt, welche die erfindungsgemäße Kompositmembran umfasst. Dabei ist die Trennvorrichtung nicht Teil des Reaktors, in dem die Epoxidierungsreaktion durchgeführt wird. Das zu trennende Reaktionsgemisch kann entweder als Ganzes aus dem Epoxidierungsreaktor entnommen und der Trennvorrichtung zugeführt werden, oder es kann kontinuierlich ein Teil des Reaktionsgemisches dem Epoxidierungsreaktor entnommen und der Trennvorrichtung zugeführt werden. Letztere Variante ist insbesondere von Vorteil, wenn die Epoxidierungsreaktion in einem kontinuierlichen Prozess durchgeführt wird. Es ist weiterhin von Vorteil, wenn dass bei der Filtration gewonnene Retentat wieder dem Epoxidierungsreaktor zugeführt wird.

In einer alternativen Ausführungsform ist die Kompositmembran Teil des Epoxidierungsreaktors. Beispielsweise können die Wände des Epoxidierungsreaktors zumindest teilweise aus der Kompositmembran gefertigt sein oder die Membran ist auf eine andere Weise direkt im Reaktionsraum untergebracht. Auf diese Weise können Epoxidierung und Filtration gleichzeitig stattfinden, so dass das vom Katalysator befreite Reaktionsgemisch enthaltend eine Mischung aus Reaktionsprodukt und Edukt kontinuierlich abgeführt werden kann. Eine entsprechende Vorrichtung zur Epoxidierung von cyclischen ungesättigten C12-Verbindungen mit Wasserstoffperoxid umfassend einen Reaktor zur Durchführung der Reaktion, dessen Wände zumindest teilweise mit einer trennaktiven Schicht aus vernetzten Siliconacrylate und/oder Polydimethylsiloxan (PDMS) und/oder Polyimid versehen sind, wird ebenfalls beschrieben.

Ebenso erfindungsgegenständlich ist die Verwendung einer solchen Vorrichtung zur gleichzeitigen Durchführung einer Epoxidierung von cyclischen ungesättigten C12-Verbindungen mit der entsprechenden Katalysatorabtrennung an der in die Wand integrierten Membran.

Ein weiterer Gegenstand der Erfindung ist ein Verfahren zur Synthese von Lactamen (erfindungsgemäßes Lactamverfahren), bei dem die epoxidierte cyclische, ungesättigte C12-Verbindungen, erhalten aus dem zuvor genannten erfindungsgemäßen Verfahren zur Abtrennung eines homogenen Katalysatorsystems, herangezogen wird.

Die epoxidierten cyclischen, ungesättigten C12-Verbindungen können im Anschluss in Anwesenheit von Wasserstoff und einem Katalysator umfassend ein Edelmetall und ein Metalloxid hydriert werden. Hierbei entsteht das entsprechende Keton sowie ggf. das Alkohol-Derivat. Sofern das Keton in einem Gemisch mit dem entsprechenden Alkohol-Derivat vorliegt, kann eine Dehydrierung des Alkohols zum Keton erfolgen. Anschließend kann das Keton oximiert werden. Im Folgeschritt kann die Beckmann-Umlagerung zum Lactam erfolgen, wobei die Umlagerung mittels Schwefelsäure oder Cyanurchlorid erfolgen kann. Die Lactame können unter Polykondensation zu Polyamiden weiterverarbeitet werden.

Die Hydrierung, die Dehydrierung, die Oximierung, die Beckmann-Umlagerung sowie die Kondensationsreaktion sind dem Fachmann bekannt.

In einer bevorzugten Ausführungsform des erfindungsgsgemäßen Lactamverfahrens wird aus Monoepoxy-cyclododecan Laurinlactam hergestellt.

Im Rahmen des bevorzugten Lactamverfahrens kann Monoepoxy-cyclododecan gewonnen werden durch folgende Reaktionsschritte: 1,3-Butadien wird durch Cyclotrimerisation zu Cyclododecatrien umgesetzt. Anschließend erfolgt eine Hydrierung zum Cyclododecen. Durch nachfolgende Epoxidierung wird das Cyclododecanepoxid erhalten.

### Beispiel 1

In einem ersten Schritt wurde eine Katalysatorlösung angesetzt. Hierzu wurden Natriumwolframat-Salz, konzentrierte Phosphorsäure sowie Wasser vorgelegt. Der pH-Wert der Lösung wurde mit 10%iger Schwefelsäure auf einen stark sauren Wert eingestellt. Im nächsten Schritt wurden in einen Rührkessel mit Heizmantel cyclisches C12-Gemsich, bestehend aus 68,5% CDEN und 31,5% Cyclododecan (CDAN) und Aliquat als Phasentransferreagenz zusammen mit der oben beschriebenen Katalysatorlösung eingefüllt. Der Reaktorinhalt wurde über Heizmantel auf etwa 80°C temperiert. Anschließend wurde eine 35%ige H₂O₂-Lösung in Wasser über eine Schlauchpumpe kontinuierlich zugeführt. Die Geschwindigkeit der H₂O₂ Zufuhr wurde dabei so eingestellt, dass die Reaktorinnentemperatur nicht über 85°C anstieg.

Nach Dosierung der H₂O₂-Lösung wurde das Gemisch mehrere Stunden bei einer Temperatur von etwa 80°C gerührt und anschließend nach Ausstellen des Rührer die Phasen getrennt. Die organische Phase wurde für den folgenden Membranfiltrationsversuch genutzt.

### Membranfiltration (organische Phase):

Ein Teil der organischen Phase wurde bei 23°C und einer transmembranen Druckdifferenz von 39 bar in einer Testzelle über der getesteten Membran gerührt.

Getestet wurde eine Polymermembran von Evonik MET Ltd. mit einer aktiven Membranfläche von ca. 50 cm².

Die aktive Trennschicht der Membran besteht aus Siliconacrylat und die Trägerschicht besteht aus Polyimid.

Nach einer Versuchsdauer von ca. 2,5 Stunden ergab sich ein Permeatfluss von 4,0 kg/(m²h). Über Proben von Retentat und Permeat wurde ein sehr hoher Wolfram-Membranrückhalt von 99,8% und ein Stickstoff-Membranrückhalt von 95,8% ermittelt.

### Beispiel 2

Ein Teil der organischen Phase aus Beispiels 1 wurde mit 55°C, einer transmembranen Druckdifferenz von 30 bar und einer Überströmung von 150 L/h über die getestete Membran geführt.

Getestet wurde eine Polymermembran von Evonik MET Ltd. mit einer aktiven Membranfläche von ca. 80 cm². Die aktive Trennschicht der Membran besteht aus Siliconacrylat und die Trägerschicht besteht aus Polyimid.

Nach einer Versuchsdauer von ca. 120 Stunden ergab sich ein Permeatfluss von 5,1 kg/(m²h). Über Proben von Retentat und Permeat wurde ein sehr hoher Wolfram-Membranrückhalt von 99,98% und ein Stickstoff-Membranrückhalt von 87,2% ermittelt.

### Beispiel 3 (kontinuierliche Epoxidierung mit nachgeschalteter Membran ohne Rückführung)

In dem Beispiel wurde die Epoxidierung der cyclischen, ungesättigten C12-Verbindungen in einer 3-stufigen Rührkesselkaskade kontinuierlich durchgeführt. Die verwendete Rührkesselkaskade beinhaltete 2 Reaktoren mit einem Nennvolumen von 5 Litern und als letzte Stufe einen Rührkessel mit einem Nennvolumen von 25 Litern. Die drei Reaktoren waren mit einem Mantel versehen und wurden über diese auf eine Temperatur von etwa 80°C temperiert.

Die cyclischen, ungesättigten C12-Verbindungen (83,8 Gew.-% CDEN und 16,2 Gew.-% CDAN), die Katalysatorkomponenten Alamin, Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor.

Das aus zwei flüssigen Phasen bestehende Reaktionsgemisch gelangte von der Kaskade in einen Phasentrennbehälter aus dem die organische Phase mittels Pumpe einer kontinuierlichen Membrananlage zugeführt wurde.

Membranfiltration (organische Phase mit MET-Membran , GMT ONF-2, Atech 1kD) Die organischen Phase wurde mit 45°C, einer transmembranen Druckdifferenz von 41,5 bar und einer Überströmung von ca. 300 L/h über die getestete Membranen MET und GMT ONF-2 geführt. Bei der zusätzlich eingesetzten Keramikmembran Atech 1kD geschah dies mit ca. 600 L/h.

Chowdhury et al. nutzen für ihre Versuche kommerziell nicht verfügbare Al₂O₃ Membranen mit Porenradien von 2,3 nm und 4,3 nm bzw. Porendurchmessern von 4,6 nm und 8,6 nm. Für das Beispiel 3 wurde deswegen ein kommerziell verfügbarer Typ Keramik - Membran mit einer besonders niedrigen Trenngrenze von 1kD genutzt, um die maximale Leistungsfähigkeit von keramischen Membranen zu verdeutlichen. Schätzungsweise hat dieser Membrantyp im Vergleich zu den von Chowdhury et. Al genutzten Membranen einen deutlich kleineren Porenradius.

Getestet wurden Polymermembranen von Evonik MET Ltd. und vom Typ ONF-2 von GMT mit einer nominalen Membranfläche von 0,6 m² bezogen auf die MET-Membran und 0,75m² bezogen auf den Typ ONF-2. Die aktive Trennschicht der Membranen besteht aus Siliconacrylat und die Trägerschicht besteht aus Polyimid. Des Weiteren wurden Atech Keramikmembranen mit einer Trenngrenze von ca. 1kD und einer Membranfläche von 540 cm² getestet

Für die oben genannten Einstellungen, ergaben sich die folgenden Prozessdaten:

| Membran | Permeatfluss | Membranrückhalt Wolfram | Membranrückhalt Stickstoff |
|---|---|---|---|
| MET-Membran | 2,2 kg/(m²*h) | 99,9 % | 73,4 % |
| ONF-2 | 2,3 kg/(m²*h) | 99,9 % | 74,5 % |
| Atech 1kD | 4,4 kg/(m²*h) | 56,2 % | 25,5 % |

Die niedrigen Rückhalte für den Typ Atech 1kD zeigen, dass mit keramischen Membranen eine technische Umsetzung zur Rückgewinnung homogener Katalysatoren aus organischen Phasen nicht sinnvoll ist.

## Patentansprüche

1. Verfahren zur Abtrennung eines homogenen Katalysatorsystems aus einem Reaktionsgemisch, bei welchem das Reaktionsgemisch zwei flüssige Phasen umfasst, wobei eine dieser Phasen an einer zumindest eine trennaktive Schicht umfassenden Membran dergestalt getrennt wird, dass das homogene Katalysatorsystem zumindest teilweise im Retentat der Membran angereichert wird, **dadurch gekennzeichnet, dass** das Reaktionsgemisch zumindest eine teilweise epoxidierte cyclische ungesättigte Verbindung mit zwölf Kohlenstoffatomen enthält, und dass die trennaktive Schicht der Membran vernetzte Siliconacrylate und/oder Polydimethylsiloxan (PDMS) und/oder Polyimid umfasst und das Katalysatorsystem ein Umsetzungsprodukt eines Derivats von Wolfram, Molybdän oder Vanadium mit einem Derivat von Phosphor in Verbindung mit einem Phasentransferreagenz umfasst, wobei das Derivat von Wolfram, Molybdän und Vanadium ausgewählt ist aus Oxiden, gemischten Oxiden, sauerstoffhaltigen Säuren, Salzen sauerstoffhaltiger Säuren, Sulfiden, Chloriden, Oxychloriden, Stearaten und den Metallcarbonylen W(CO)₆ und Mo(CO)₆, wobei das Derivat von Phosphor ausgewählt ist aus Oxiden, Sauerstoffsäuren, Salzen einer Sauerstoffsäure, Sulfiden, Chloriden, Oxychloriden oder Fluoriden, und wobei das Phasentransferreagenz eine tertiäre oder quartäre Ammoniumverbindung ist.

2. Verfahren nach Anspruch 1, **dadurch gekennzeichnet, dass** die zwei Phasen eine organische Phase enthaltend die zumindest teilweise epoxidierte cyclische, ungesättigte Verbindung mit zwölf Kohlenstoffatomen sowie eine wässrige Phase umfasst.

3. Verfahren nach Anspruch 2, **dadurch gekennzeichnet, dass** die wässrige Phase Wasserstoffperoxid enthält.

4. Verfahren nach einem der vorhergehenden Ansprüche, **dadurch gekennzeichnet, dass** es sich bei der cyclischen ungesättigten Verbindung mit zwölf Kohlenstoffatomen um Cyclododecen handelt.

5. Verfahren nach einem der vorhergehenden Ansprüche, **dadurch gekennzeichnet, dass** das Katalysatorsystem Natriumwolframat und Phosphorsäure umfasst.

6. Verfahren nach einem der vorhergehenden Ansprüche, **dadurch gekennzeichnet, dass** die quartäre Ammoniumverbindung ein Esterquat der Formel (I) ist 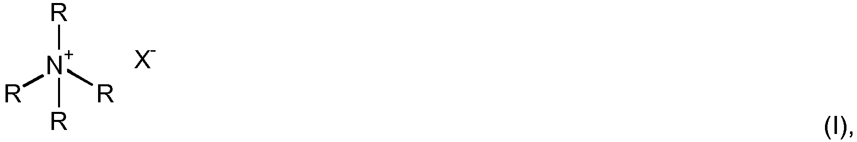 wobei die Substituenten R unabhängig voneinander gleiche oder verschiedene Alkyl- oder Alkoholgruppen mit 1 bis 4 Kohlenstoffatomen darstellen und mindestens einer der Substituenten R eine Alkoholgruppe mit 1 bis 4 Kohlenstoffatomen darstellt, die mit einer gesättigten oder ungesättigten Fettsäure mit 1 bis 30 Kohlenstoffatomen verestert ist, und wobei X⁻ für ein Gegenanion steht.

7. Verfahren nach einem der vorhergehenden Ansprüche, **dadurch gekennzeichnet, dass** die quartäre Ammoniumverbindung ein Ester einer quartären Ammoniumverbindung gemäß den Formeln Formeln (II) oder (III) 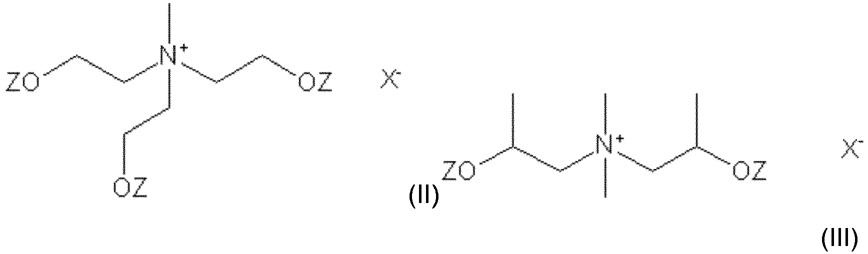 mit Z gleich einer oder mehreren, gesättigten oder ungesättigten Fettsäuren mit 1 bis 30 Kohlenstoffatomen ist, wobei X⁻ für ein Gegenanion steht.

8. Verfahren nach einem der vorhergehenden Ansprüche, **dadurch gekennzeichnet, dass** es sich bei den Siliconacrylaten um Verbindungen der Formel IV handelt 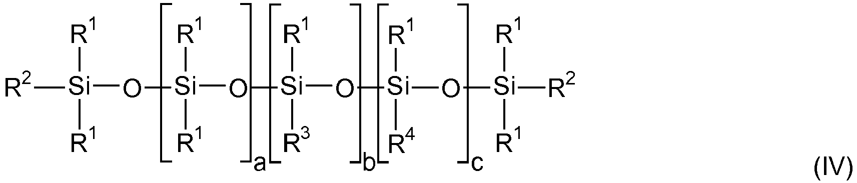 wobei
a = 1 bis 500, b = 1 bis 25 und c = 0 bis 20 ist,
die Substituenten R¹ unabhängig voneinander gleiche oder verschiedene Alkyl- oder Arylgruppen mit 1 bis 30 Kohlenstoffatomen darstellen, die gegebenenfalls mindestens eine Ether-, Ester-, Epoxy- und/oder Hydroxygruppe tragen können, die Substituenten R² unabhängig voneinander gleiche oder verschiedene Substituenten aus der Gruppe R¹, R³ und R⁴ darstellen,
die Substituenten R³ unabhängig voneinander gleiche oder verschiedene Acrylatgruppen der Formeln V oder VI darstellen 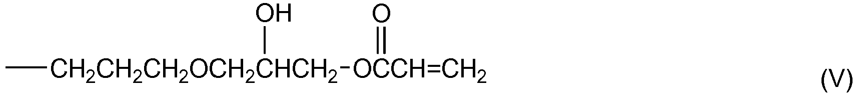 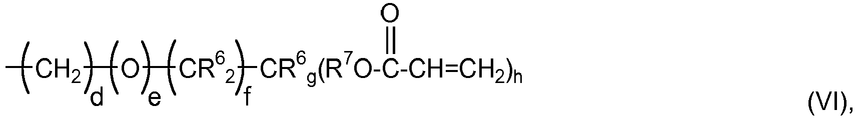
wobei d = 0 bis 12, e = 0 bis 1, f = 0 bis 12, g = 0 bis 2, h = 1 bis 3 und g + h = 3 ist,
die Substituenten R⁶ unabhängig voneinander gleiche oder verschiedene Alkyl- oder Arylgruppen mit 1 bis 30 Kohlenstoffatomen oder Wasserstoff darstellen,
die Gruppen R⁷ gleiche oder verschiedene zweibindige Kohlenwasserstoffreste, bevorzugt -CR⁶₂-, insbesondere CH₂, darstellen,
die Substituenten R⁴ unabhängig voneinander gleiche oder verschiedene Polyethergruppen der Formel (VII) darstellen 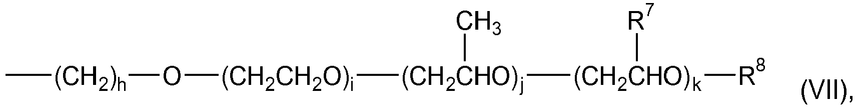
wobei i = 0 bis 12, j = 0 bis 50, k = 0 bis 50 und I = 0 bis 50 ist,
die Substituenten R⁸ gleiche oder verschiedene Alkyl- oder Arylgruppen mit 2 bis 30 Kohlenstoffatomen darstellen, und
R⁹ eine Alkyl-, Aryl- oder Acylgruppe mit 2 bis 30 Kohlenstoffatomen oder Wasserstoff darstellt.

9. Verfahren nach einem der vorhergehenden Ansprüche, **dadurch gekennzeichnet, dass** die trennaktive Schicht auf einem Trägermaterial ausgewählt aus Polyacrylnitril, Polyimid, Polyetheretherketon, Polyvinylidenfluorid, Polyamid, Polyamidimid, Polyethersulfon, Polybenzimidazol, sulfoniertes Polyetherketon, Polyethylen, Polypropylen oder Mischungen daraus aufgebaut ist.

10. Verfahren nach einem der vorhergehenden Ansprüche, **dadurch gekennzeichnet, dass** bei der Trennung ein Transmembrandruck von 5 bis 80 bar angelegt wird.

11. Verfahren nach einem der vorhergehenden Ansprüche, **dadurch gekennzeichnet, dass** die Trennung bei einer Temperatur von 25 bis 160 °C durchgeführt wird.

12. Verwendung einer Vorrichtung umfassend einen Reaktor zur Durchführung der Reaktion, **dadurch gekennzeichnet, dass** die Wände des Reaktors zumindest teilweise mit einer trennaktiven Schicht aus vernetzten Siliconacrylate und/oder Polydimethylsiloxan (PDMS) versehen sind, zur gleichzeitigen Durchführung einer Epoxidierung von cyclischen, ungesättigten C12-Verbindungen mit Wasserstoffperoxid und einem Verfahren nach einem der Ansprüche 1 bis 11.

## Claims

1. Method of removing a homogeneous catalyst system from a reaction mixture wherein the reaction mixture comprises two liquid phases wherein a membrane comprising at least one separation-active layer separates one of these phases in such a way that the homogeneous catalyst system is concentrated in the membrane retentate at least to an extent, **characterized in that** the reaction mixture comprises at least one partially epoxidized cyclic unsaturated compound comprising twelve carbon atoms and the membrane separation-active layer comprises crosslinked silicone acrylates and/or polydimethylsiloxane (PDMS) and/or polyimide, and the catalyst system comprises a reaction product of a tungsten, molybdenum or vanadium derivative with a phosphorus derivative in combination with a phase transfer reagent,
wherein the tungsten, molybdenum and vanadium derivative is selected from oxides, mixed oxides, oxoacids, salts of oxoacids, sulphides, chlorides, oxychlorides, stearates and the metal carbonyls W(CO)₆ and Mo(CO)₆,
wherein the phosphorus derivative is selected from oxides, oxoacids, salts of an oxoacid, sulphides, chlorides, oxychlorides or fluorides, and
wherein the phase transfer reagent is a tertiary or quaternary ammonium compound.

2. Method according to Claim 1, **characterized in that** the two phases comprise an aqueous phase and an organic phase comprising the at least partially epoxidized cyclic unsaturated compound having twelve carbon atoms.

3. Method according to Claim 2, **characterized in that** the aqueous phase comprises hydrogen peroxide.

4. Method according to any preceding claim, **characterized in that** the cyclic unsaturated compound having twelve carbon atoms is cyclododecene.

5. Method according to any preceding claim, **characterized in that** the catalyst system comprises sodium tungstate and phosphoric acid.

6. Method according to any preceding claim, **characterized in that** the quaternary ammonium compound is an esterquat of formula (I) 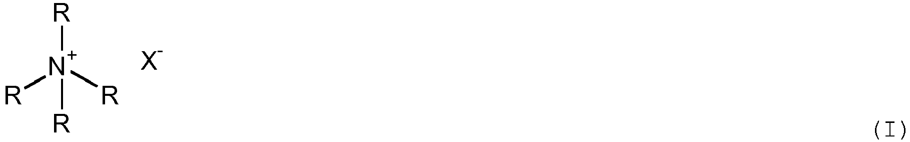 where the substituents R are independently identical or different alkyl or alcohol groups comprising from 1 to 4 carbon atoms and at least one of the substituents R is an alcohol group which comprises from 1 to 4 carbon atoms and which has been esterified with a saturated or unsaturated fatty acid comprising from 1 to 30 carbon atoms and X⁻ is a counteranion.

7. Method according to any preceding claim, **characterized in that** the quaternary ammonium compound is a quaternary ammonium compound ester according to formulae (II) and (III) 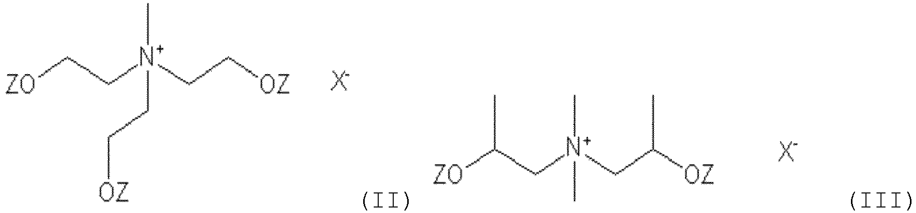 where Z denotes one or more saturated or unsaturated fatty acids comprising from 1 to 30 carbon atoms and X⁻ is a counteranion.

8. Method according to any preceding claim, **characterized in that** the silicone acrylates are compounds of formula IV 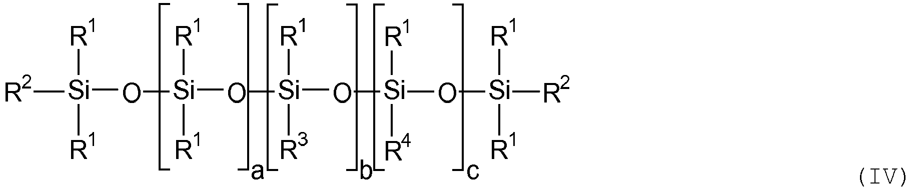 where
a = from 1 to 500, b = from 1 to 25 and c = from 0 to 20,
the substituents R¹ are independently identical or different alkyl or aryl groups which comprise from 1 to 30 carbon atoms and which may bear at least one ether, ester, epoxy and/or hydroxy group,
the substituents R² are independently identical or different substitutents selected from the group consisting of R¹, R³ and R⁴,
the substituents R³ are independently identical or different acrylate groups of formulae V or VI 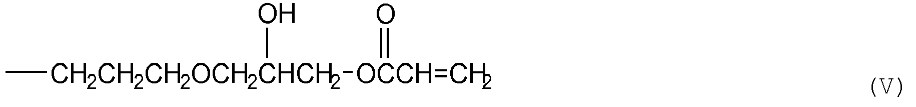 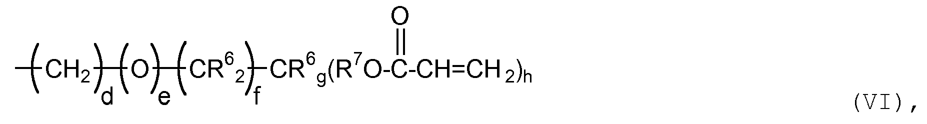
where d = from 0 to 12, e = from 0 to 1, f = from 0 to 12, g = from 0 to 2, h = from 1 to 3 and g + h = 3,
the substituents R⁶ are independently identical or different alkyl or aryl groups comprising from 1 to 30 carbon atoms or hydrogen,
the groups R⁷ are identical or different divalent hydrocarbon radicals, preferably -CR⁶₂- and in particular CH₂,
the substituents R⁴ are independently identical or different polyether groups of formula (VII) 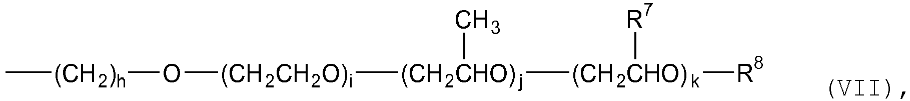
where i = from 0 to 12, j = from 0 to 50, k = from 0 to 50 and 1 = from 0 to 50,
the substituents R⁸ are identical or different alkyl or aryl groups comprising from 2 to 30 carbon atoms and
R⁹ is an alkyl, aryl or acyl group comprising from 2 to 30 carbon atoms or hydrogen.

9. Method according to any preceding claim, **characterized in that** the separation-active layer is arranged on a carrier material selected from polyacrylonitrile, polyimide, polyether ether ketone, polyvinylidene fluoride, polyamide, polyamide-imide, polyethersulphone, polybenzimidazole, sulphonated polyether ketone, polyethylene, polypropylene or mixtures thereof.

10. Method according to any preceding claim, **characterized in that** the separation is carried out with an applied transmembrane pressure of from 5 to 80 bar.

11. Method according to any preceding claim, **characterized in that** the separation is carried out at a temperature of from 25°C to 160°C.

12. Use of an apparatus comprising a reactor for carrying out the reaction, **characterized in that** the walls of the reactor are at least partially furnished with a separation-active layer of crosslinked silicone acrylates and/or polydimethylsiloxane (PDMS), for carrying out simultaneously an epoxidation of cyclic unsaturated C12 compounds with hydrogen peroxide and a method according to any of Claims 1 to 11.

## Revendications

1. Procédé de séparation d'un système catalytique homogène d'un mélange réactionnel, selon lequel le mélange réactionnel comprend deux phases liquides, une de ces phases étant séparée sur une membrane comprenant au moins une couche à activité de séparation de telle sorte que le système catalytique homogène s'accumule au moins en partie dans le rétentat de la membrane, **caractérisé en ce que** le mélange réactionnel contient au moins un composé insaturé cyclique partiellement époxydé contenant douze atomes de carbone, et **en ce que** la couche à activité de séparation de la membrane comprend des acrylates de silicone réticulés et/ou du polydiméthylsiloxane (PDMS) et/ou du polyimide, et le système catalytique comprend un produit de réaction d'un dérivé de tungstène, de molybdène ou de vanadium avec un dérivé de phosphore en liaison avec un réactif de transfert de phase,
le dérivé de tungstène, de molybdène et de vanadium étant choisi parmi les oxydes, les oxydes mixtes, les acides oxygénés, les sels d'acides oxygénés, les sulfures, les chlorures, les oxychlorures, les stéarates et les carbonyles de métaux W(CO)₆ et Mo(CO)₆,
le dérivé de phosphore étant choisi parmi les oxydes, les acides oxygénés, les sels d'un acide oxygéné, les sulfures, les chlorures, les oxychlorures ou les fluorures, et
le réactif de transfert de phase étant un composé d'ammonium tertiaire ou quaternaire.

2. Procédé selon la revendication 1, **caractérisé en ce que** les deux phases comprennent une phase organique contenant ledit au moins un composé insaturé cyclique au moins partiellement époxydé contenant douze atomes de carbone, ainsi qu'une phase aqueuse.

3. Procédé selon la revendication 2, **caractérisé en ce que** la phase aqueuse contient du peroxyde d'hydrogène.

4. Procédé selon l'une quelconque des revendications précédentes, **caractérisé en ce que** le composé insaturé cyclique contenant douze atomes de carbone consiste en du cyclododécène.

5. Procédé selon l'une quelconque des revendications précédentes, **caractérisé en ce que** le système catalytique comprend du tungstate de sodium et de l'acide phosphorique.

6. Procédé selon l'une quelconque des revendications précédentes, **caractérisé en ce que** le composé d'ammonium quaternaire est un esterquat de formule (I) 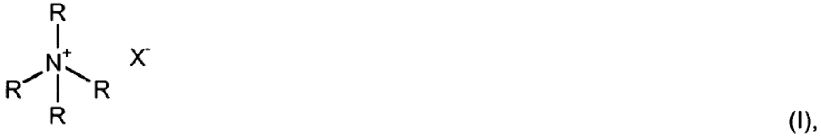 dans laquelle les substituants R représentent indépendamment les uns des autres des groupes alkyle ou alcool de 1 à 4 atomes de carbone identiques ou différents, et au moins un des substituants R représente un groupe alcool de 1 à 4 atomes de carbone qui est estérifié avec un acide gras saturé ou insaturé de 1 à 30 atomes de carbone, et dans laquelle X⁻ représente un contre-anion.

7. Procédé selon l'une quelconque des revendications précédentes, **caractérisé en ce que** le composé d'ammonium quaternaire est un ester d'un composé d'ammonium quaternaire selon la formule (II) ou (III) 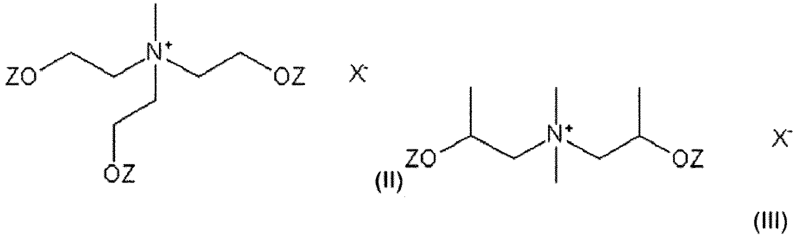 dans lesquelles Z représente un ou plusieurs acides gras saturés ou insaturés de 1 à 30 atomes de carbone, X⁻ représentant un contre-anion.

8. Procédé selon l'une quelconque des revendications précédentes, **caractérisé en ce que** les acrylates de silicone sont des composés de formule IV 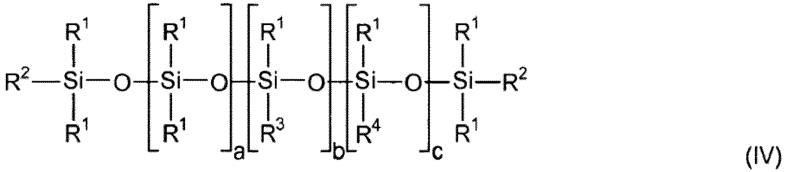 dans laquelle
a = 1 à 500, b = 1 à 25 et c = 0 à 20,
les substituants R¹ représentent indépendamment les uns des autres des groupes alkyle ou aryle de 1 à 30 atomes de carbone identiques ou différents, qui peuvent éventuellement porter au moins un groupe éther, ester, époxy et/ou hydroxy,
les substituants R² représentent indépendamment les uns des autres des substituants identiques ou différents du groupe constitué par R¹, R³ et R⁴,
les substituants R³ représentent indépendamment les uns des autres des groupes acrylate identiques ou différents de formule V ou VI 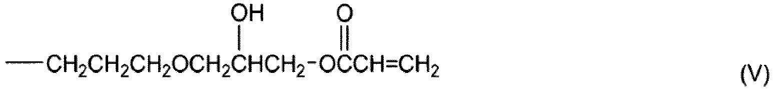 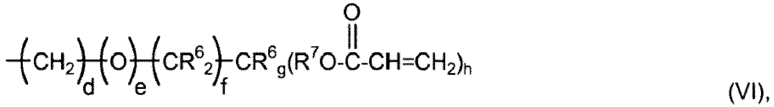
dans lesquelles d = 0 à 12, e = 0 à 1, f = 0 à 12, g = 0 à 2, h = 1 à 3, et g + h = 3,
les substituants R⁶ représentent indépendamment les uns des autres des groupes alkyle ou aryle de 1 à 30 atomes de carbone identiques ou différents, ou l'hydrogène, les groupes R⁷ représentent des radicaux hydrocarbonés bivalents identiques ou différents, de préférence -CR⁶₂-, notamment CH₂,
les substituants R⁴ représentent indépendamment les uns des autres des groupes polyéther identiques ou différents de formule (VII) 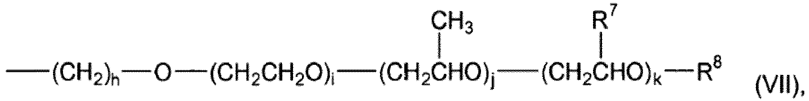
dans laquelle i = 0 à 12, j = 0 à 50, k = 0 à 50 et 1 = 0 à 50,
les substituants R⁸ représentent des groupes alkyle ou aryle de 2 à 30 atomes de carbone identiques ou différents, et
R⁹ représente un groupe alkyle, aryle ou acyle de 2 à 30 atomes de carbone ou l'hydrogène.

9. Procédé selon l'une quelconque des revendications précédentes, **caractérisé en ce que** la couche à activité de séparation est à base d'un matériau support choisi parmi le polyacrylonitrile, le polyimide, la polyéther-éther-cétone, le polyfluorure de vinylidène, le polyamide, le polyamide-imide, la polyéther-sulfone, le polybenzimidazole, la polyéther-cétone sulfonée, le polyéthylène, le polypropylène ou les mélanges de ceux-ci.

10. Procédé selon l'une quelconque des revendications précédentes, **caractérisé en ce qu'**une pression transmembranaire de 5 à 80 bar est appliquée lors de la séparation.

11. Procédé selon l'une quelconque des revendications précédentes, **caractérisé en ce que** la séparation est réalisée à une température de 25 à 160 °C.

12. Utilisation d'un dispositif comprenant un réacteur pour la réalisation de la réaction, **caractérisée en ce que** les parois du réacteur sont au moins partiellement munies d'une couche à activité de séparation en acrylates de silicone réticulés et/ou en polydiméthylsiloxane (PDMS), pour la réalisation simultanée d'une époxydation de composés en C12 insaturés cycliques avec du peroxyde d'hydrogène et d'un procédé selon l'une quelconque des revendications 1 à 11.
